# EUROPEAN PATENT SPECIFICATION

(11) **EP 3 445 456 B1**
(45) Date of publication and mention of the grant of the patent: **19.05.2021**
(21) Application number: 16719193.1
(22) Date of filing: 18.04.2016
(51) Int. Cl.: A61K 8/19, A61Q 19/08, A61K 33/00

(54) **COSMETIC COMPOSITION COMPRISING WATER DEPLETED IN 2H AND 18O ISOTOPS**
KOSMETISCHE ZUSAMMENSETZUNG MIT AN 2H- UND 18O-ISOTOPEN ABGEREICHERTEM WASSER
COMPOSITION COSMÉTIQUE COMPRENANT DE L'EAU APPAUVRIE EN ISOTOPES 2H ET 18O

(43) Date of publication of application: 27.02.2019
(73) Proprietor: Medena AG, 8910 Affoltern a. A. (CH)
(72) Inventor: MAYER, Wolfgang, 8006 Zurich (CH); KORKINA, Liudmila, Moscow 117513 (RU)
(74) Representative: Lusuardi, Werther
(86) International application number: PCT/CH2016/000068
(87) International publication number: WO 2017/181296

(56) References cited:
- WO-A2-2005/065631
- JP-A- 2004 231 627
- RU-C2- 2 287 318
- US-A1- 2008 138 439
- US-A1- 2008 160 100

## Description

The invention relates to a cosmetic composition according to the preamble of claim 1.

Water is made of hydrogen and oxygen, but both of these elements have more than one stable naturally occurring isotope. The most abundant hydrogen isotope has an atomic mass number of 1, but the mass number of 2 (deuterium and represented by the symbol D or ²H) is present in small quantities (0.0156% or 156ppm of all hydrogen atoms). D is present in surface water in the form of HDO (semi-heavy water) or D₂O (heavy water) at a total concentration of 16.8 mmol/L or 150 ppm. The ratio of two stable isotopes of hydrogen strongly influences chemical and physical properties of water.

The most abundant oxygen isotope has a mass number of 16, but the 18-0 isotope is present at about 0.2% and there is also a tiny amount of 17-0.
The natural isotopic composition of water is not uniform. This is because of differences in volatility between water molecules containing different isotopes. When water vaporizes, the vapor is depleted in the heavier isotopes: the heavier isotope is the more depleted of it the vapor is. The opposite occurs when water condenses from the atmosphere; the rain or snow has more of the heavy isotopes, leaving lighter water vapor in the atmosphere.
Because of these effects, fresh waters on Earth vary relatively widely in their isotopic composition. In temperate climates, fresh water is about 4% depleted in deuterium and 2% enriched in 18-0 compared to ocean water. In the Polar Regions, the D depletion can reach 40% while 18-0 enrichment can be up to 20%.
The isotopic composition of deep offshore ocean water is remarkably uniform across the Earth. Thus, the ocean water provides an isotopic ZERO "standard", which is easily reproducible and against which other waters could be compared. Throughout geologic time the oxygen mass budget was likely conserved within the Earth system reservoirs, but hydrogen's was not, as it can escape to space. For example, Archaean oceans were depleted in deuterium by at most 25.5 ‰, but oxygen isotope ratios were comparable to modern oceans.

### Peculiarities of physical and chemical properties of natural heavy isotopes of water

The physical properties of heavy water, water enriched with the deuterium and heavy-oxygen isotopes, are well-known. The boiling and freezing points of such water are shifted from the relevant points of normal water. The dilution of D-depleted water (2 ppm) by heavier water up to 20 ppm elevates kinematic viscosity and surface tension whereas it does not affect water density. On contrast, an increase in temperature results in decreased values of viscosity and surface tension. Hence water depleted from heavy oxygen and hydrogen isotopes exerts physical chemical properties of heated water, which usually solubilize more organic and non-organic molecules and better penetrates through porous materials.

### Abundance of heavy water isotopes in living organisms

Water is an essential requirement for life. It is the largest constituent of all living organisms. For example, the human body consists of 60-70% water, the content depends on the age: the older organism is the more it is deprived from water. Intra- and extracellular water plays numerous physiological roles, providing an appropriate medium for biochemical reactions in cells, cell-to-cell interactions, and cellular responses to biotic and abiotic stresses. Living organisms get a water supply from environmental water and food they consume or get in touch with as well as from atmospheric water (humidity) penetrating through their skin and other exposed lining epithelia (oral, eye, ear, nasal, etc.) or absorbed by their surface hair. In general, isotopic ratios of oxygen and hydrogen within an organism are largely defined by those of consumed/absorbed water. As compared to other biologically important micro elements and organic substances in human body, such as Ca, Mg, K and glucose, the normal blood serum concentrations of hydrogen and oxygen isotopes are quite high: Ca - 2,24-2,74 mM; Mg - 0,75-1,20 mM; K - 3,5-5,1 mM; glucose - 3,3-6,1 mM; 2-H - 12-14 mM; and 18-0 - 100-110 mM. However, isotopic distribution between different cells/tissues of a multicellular organisms (humans, animal, and higher plants) differs a lot from one cell type to another and from one tissue to another. This fractionation of, first of all, ¹⁸O and ¹⁶O water isotopes as well as ²H and ¹H isotopes takes place because numerous biochemical reactions in plant, microbial, animal or human organisms can discriminate between water isotopes. Due to such discrimination, levels of ¹⁸O in human and animal organisms tend to be greater than those in consumed water and food. Moreover, ¹⁸O is highly concentrated in the skin, epithelial linings, hair, nails, and bones.

Regarding ²H organism's levels, they are slightly lower than those in consumed water and food, and humidity. The distribution of ²H is favorable for soft tissues while blood plasma is deprived of the isotope. It is assumed that the hydrogen isotopic compositions of biomolecules strongly reflect both the δD values of their sources and isotopic fractionations during biochemical redox reactions as well as hydrogen-exchange reactions. Hydrogens removed during dehydrogenation and those incorporated during hydrogenation are substantially depleted in D due to the large kinetic isotope effects associated with the enzymatic biochemical reactions.

### Biochemical fractionation of heavy isotopes of water

Oxidative transformations using molecular oxygen are widespread in nature. Biochemical reactions with the evidence of prevalent ¹⁸O capture/inclusion are the following: protein synthesis of collagen and keratins, extra cellular matrix (ECM) polysaccharide synthesis, and CaCO₃ mineral synthesis in bones. In addition, ¹⁸O affects kinetic parameters of definite redox reactions shifting their equilibrium and changing reversible reactions to irreversible ones. The most spectacular example is metal-catalyzed redox reactions in the active sites of peroxidases and catalase, kinetic rate and efficiency of which as well as oxygen coordination to transition metal and cleavage of the O-O bond depend on the oxygen isotope used. Oxygen-18 isotope is intensively exhaled in the form of CO₂ by patients suffering from pre-diabetes and type 2 diabetes mellitus that led to suggestion that this isotope is linked to altered activity of lung carbonic anhydrase enzyme in such patients. The same isotope in human breath CO₂ is a marker of *H.pilori* viral infection.
Hydrogen is an essential element of bioactive organic molecules, and its isotope effects are commonly remarkable. The biosynthetic fractionation of hydrogen strongly depends on biological species. For example, practically all plants, terrestrial and aquatic, are more depleted in D as compared to environmental surface water used for the plant metabolism due to a strong fractionation during photosynthesis. In general, both plant-derived proteins and lipids contain lower levels of D than in the water which nourishes the plants. At the same time, seaweeds have larger isotopic fractionation and they are more depleted in D than terrestrial and fresh water plants. Lipids (fatty acids and esters) isolated from marine seaweed contain much less D than terrestrial and freshwater plants grown in similar environment (the ration D/H equal to 155ppm for Japanese/Thailand area was found diminished to approx. 100ppm in lipids derived from seaweed (*Gelidium japonicum* and *Undaria pinnatifida*). The lowest levels of D have been found in phytol, a sterol-like lipid derived from isoprenoid biosynthetic pathway in higher plants and in several other metabolites of the same molecular process, such as sesquiterpens, squalene, and beta-sitosterol. Isotopic fractionation of H and D occurs during chlorophylls synthesis through tricarboxylic acid cycle as well.

Collectively, lipid soluble active ingredients from marine organisms, fist of all seaweed, isoprenoid lipids, and polyphenols from some terrestrial plants are highly deprived with heavy isotopes, such as D, ¹⁸O and ¹³C. Being used in combination with deuterium and oxygen-18 depleted water, they are the safest and the most biologically efficient topically applied compositions.

### Biological effects of heavy isotopes of water

Although the effect of deuterium enriched water due to D isotopic effect at an elevated concentration of D in biological systems has been investigated the significance of naturally occurring D concentration has yet to be addressed. Variations in concentrations of D influence cellular functions and growth because in living organisms the D/H ratio changes during cell cycle and D seems to have an essential role in signal transduction of cell cycle control. It has been quickly found that highly enriched deuterium oxide ("heavy water") negatively affects growth and well-being of many organisms. Large amounts of deuterium in water were found to reduce protein and nucleic acids synthesis, disturb cell division and alter cellular morphology. High concentrations of deuterium were proven toxic to higher organisms, although some bacteria are able to adapt to grow in almost pure heavy water. Water containing 100% D causes a block of hydrogen ion transportation and as a consequence significant alterations in enzyme activities, cell metabolism, and physiology occurs.

A link between ageing and D is well established. D₂O concentrations exceeding the natural level resulted in numerous adverse effects: (a) increased viral mutation rates; (b) deuteration of synthetic oestrogen hormones weakened its oestrogenic properties; (c) deuterated enzymes exhibited conformational changes, affecting their active sites; (d) the skin became enriched in deuterium along a temporal ageing axis; and (e) reduced the lifespan of mice.

There have been much fewer reports on the effect of other heavy stable isotopes in biology. Since only high enrichments with heavy isotopes have produced statistically significant alterations and this enrichment is extremely difficult and expensive for ¹⁸O, there were practically no experimental or clinical studies done with the heavy isotope. However, ¹⁸O reactions with biologically important molecules, such as proteins (enzymes and receptors are among them), nucleic acids, polysaccharides, and lipids allow to suggest that even slight deviations from natural levels of ¹⁸O in an organisms, definite tissues, and cells could seriously alter their structures and functions. That is the case of skin, hair, nails, and lining epithelia which are in constant contact with environmental water, man-made water-containing topical products, and are also the targets for orally consumed water in beverages and food.

### Heavy isotopes of water and skin

It is of common knowledge that skin is a unique physical, chemical, and biological barrier, which protects the entire organism against external invasions. While the physical protection is secured by a particularly compact structure of *stratum corneum* and epidermis in general, the biological defense depends on a complex skin-located immune system. The cutaneous chemical barrier consists of numerous enzymes and non-enzymatic molecules able of reacting with and facilitating the metabolism of low molecular weight foreign substances. The topically applied dermatological drugs, skin care products, cosmetics, aromatic oils, and perfumes as well as non-intentional contact with environmental hazards such as organic toxins, solar UV irradiation, dust particles, tobacco smock, heavy metals, etc. may affect (activate or inactivated) the barrier properties of the skin making it highly responsive to unfriendly environment and to internal signaling from organism. As a consequence, skin interaction with physical, biological, and chemical agents may bring locally either health effects (anti-inflammatory, wound healing, angiogenic, chemotherapeutic, or anti-microbial) or undesirable adverse effects (skin sensitization, skin carcinogenesis, or photo-toxic reactions in the skin). It is of great importance that water loss from the skin and lining epithelia by passive evaporation (trans-epithelial water loss) as well as by active transpiration (sweating, inflammatory exsudate) is highly intensified in ageing, stressed (heat stress, psychological stress, mechanical stress, chemical stress, endocrine stress, solar irradiation-induced stress, etc.), and ailing skin. Taking into account the general principle of heavy isotopes retention during evaporation-transpiration process, one can assume that ¹⁸O and ²H concentrations will be highly increased in such suffering skin/epithelia. Any mean to balance the water isotope ratio at the epithelial level will inevitably lead to normalization of its metabolism, cell growth and barrier functions.

As a current trend, cosmetic/cosmeceutical products contain rather big quantities from 50% to 70% and even 90% of water (aqueous sprays, water/lipid creams, gels, foams, non-alcohol lotions, perfumes, face and body washes etc.). According to International regulations for manufacturers of cosmetics, the quality of water should be very high. Therefore the use of bidistilled and microbe-free (sterilized) water is strictly required.

Various cosmetic preparations are known which for which water enriched in ¹H and ¹⁶O isotopes (up to 99.76-99.80% of total water content) was used. Therefore the content of these known cosmetic preparations for ²H ranged from 111 to 133ppm and the content of ¹⁸O ranged from 1481 to 1778ppm. The high content of water enriched in ¹H and ¹⁶O was probably intended to obtain isotopic homogeneity in order to presumably improve quality of theses cosmetic preparations but without success.

From US 2008/138439 a cosmetic composition is known enriched with ¹H₂¹⁶O but which is silent to the content of ¹⁸O and of ²H as such.

From US 2008/160100 a method for stimulating hair growth and regeneration in a mammal in need thereof is known, however there is no disclosure regarding the amount of single residual isotopologes being present in the composition.
From RU 2 287 318 means for the care of the skin, hair, nails and oral cavity of a person is known.
From WO 2005/065631 a cosmetic and hygiene product is known which comprises 5 to 96% by weight of deuterium-depleted water (ASD).
Therefore none of the cited prior art documents is disclosing minimum and maximum amounts of the isotopes of ¹⁸O and of ²H according to the present invention.

What is therefore needed is an improved cosmetic composition.

It is an object of the invention to provide a cosmetic composition with improved cosmetic effects having specifically defined low ranges of the isotopes of ¹⁸O and of ²H. It is a further object of the invention to provide a use of said cosmetic composition.

The invention solves the posed problem with a cosmetic composition comprising the features of claim 1 and a non-therapeutic use of that cosmetic composition comprising the features of claim 12.

Most surprisingly it was found that the presence of a certain amount of heavy isotopes is essential for cellular growth and differentiation. The advantages of the invention are normalization of isotope ratio in biological structures and molecules of epidermis/epithelia due to isotopic exchange, consequent normalization of epidermal/epithelial functions and structure, enhanced bioavailability/biological efficacy of active ingredients-components of the topical preparations, and enhanced microbiological, toxicological, and heavy metal-dependent safety of the final topical composition.

Further advantages of the invention are the following:
(i) to provide optimal cell growth and differentiation;
(ii) to provide optimal rates of chemical reactions (reduction/oxidation and dehydrogenation);
(iii) to provide optimal conditions for activities of several enzymes (redox-dependent, H-channels, ATPases, kinases);
(iv) to provide optimal interactions between receptors and ligands which greatly depend on hydrogen bonds and reduction/oxidation of SH-groups; and
(v) to increase solubility of active ingredients/drugs, to increase stability of final compositions containing high concentrations of active principles, to enhance penetration of active ingredients through epithelial/epidermal barrier, to protect unstable active principles against non-enzymatic destruction and enzymatic metabolic transformations, to increase bioavailability of active ingredients/drugs, and to increase biological interaction between the compositions and human tissues/organs.

A further object of the invention is the use of water depleted physically from ¹⁸O and ²H and active substances of plant origin naturally depleted from these heavy isotopes for topical compositions targeting mainly ageing, stressed, and ailing skin, hair, nails, and lining epithelia as well as highly sensitive skin of children, pregnant women, and immune compromised subjects. The use of ¹⁸O and ²H depleted water/compositions for topical use in animals is also a subject of the present invention.

Further advantageous embodiments of the invention can be commented as follows:
The water in the cosmetic composition preferably contains at most 1050 ppm of ¹⁸O and at most 85 ppm of ²H. More preferably the water in the cosmetic composition contains at most 1000 ppm of ¹⁸O and at most 80 ppm of ²H.

The cosmetic composition purposefully comprises water in an amount of 50 to 90 weight-%, preferably in an amount of 55 to 70 weight-%.

The water in the cosmetic composition preferably contains at least 600 ppm of ¹⁸O. Further the water in the cosmetic composition preferably contains at least 30 ppm of ²H.

In a further embodiment the cosmetic composition is essentially free of alcohols. In that case the water purposefully comprises more than 675 ppm of ¹⁸O, preferably more than 750 ppm of ¹⁸O. And further the water comprises purposefully more than 55 ppm of ²H, preferably more than 65 ppm of ²H.

In a further embodiment the water is derived from plants comprising substances of cosmetic importance, in particular lipids, polysaccharides, proteins, amino acids and polyphenols, which are depleted from heavy hydrogen and heavy oxygen isotopes during their bio-synthesis and isotopic fractionation at definitive steps of the biosynthesis.

The cosmetic composition may further comprise a non-medicinal active principle derived from plant extracts or purified plant-derived agents, in particular polyphenols like flavonoids. Many plant-derived active agents used in cosmetics have low solubility in polar solvents such as water or ethanol. Their solubility could be increased by heating, long stirring or adding apolar solvents. However, stability of such "forced" solutions is rather low and an active agent could form sediments or the solution could fractionate to polar and non-polar phases. Increased solubility allows reaching higher stable concentrations of ingredients in aqueous solutions. It was found that this phenomenon mainly depends on the more regular three-dimensional structure of light water and on the strength of hydrogen bonds established between water-water and water-solubilized substance molecules. Solubility positively correlates with the content of light isotopes in water according to the invention.

Preferably the water used in the cosmetic composition has:
- a weight concentration of residue after evaporation of no more than 5 mg/dm³;
- a weight concentration of ammonia and ammonium salts of no more than 0.02 mg/dm³;
- a weight concentration of nitrates of no more than 0.2 mg/dm³;
- a weight concentration of sulfates of no more than 0.5 mg/dm³;
- a weight concentration of chlorides of no more than 0.02 mg/dm³;
- a weight concentration of aluminum of no more than 0.05 mg/dm³;
- a weight concentration of iron ions of no more than 0.05 mg/dm³;
- a weight concentration of calcium ions of no more than 0.8 mg/dm³;
- a weight concentration of copper ions of no more than 0,02 mg/dm³;
- a weight concentration of lead ions of no more than 0.05 mg/dm³;
- a weight concentration of zinc ions of no more than 0.2 mg/dm³;
- a weight concentration of KMnO₄(O)-reducing substances of no more than 0.08 mg/dm³;

In a further embodiment the water used in the cosmetic composition has a pH value of 5.4 - 6.6. The water may have a specific conductivity at 20 °C, S/m, of no more than 5.10⁻⁴.

In a further embodiment the water has a deuterium content of no more than 50 ppm.

The cosmetic composition according to the invention may be used for the following applications:
- topical administration to human skin and skin appendages, preferably to hair or nails;
- topical administration to human or animal lining epithelia, preferably orally, nasally, vaginally, uretrally, anally or for the eye or ear;
- for topical administration to attenuate epithelial cell inflammatory responses to biotic and abiotic stresses; or
- topical administration to promote cellular longevity of skin cells resulting in skin rejuvenation;

The cosmetic composition according to the invention can also be used together with a pharmaceutically active drug. The advantages are an enhancement of the penetration of the drug through epidermal/epithelial barrier; and an enhancement of the bioavailability of the pharmaceutically active drug for epidermal/epithelial cell metabolism.

Enhanced penetration through epidermal/epithelial barrier allows achieving active concentration of ingredients in different skin layers/at different skin depth. Otherwise, active principles could remain at the outmost skin layer of non-vital skin cells keratinocytes where they cannot exert their expected biological action.
Active principles of topical compositions target definite skin cells or extracellular matrix molecules. Reaching their targets, they can exert their expected biological action-interaction with molecular processes or cell-to-cell communications or other cell functions (bioavailability).
The cosmetic preparations according to the invention have decreased surface tension as compared with the same compositions prepared with natural distilled water. It was found that the compositions with De-O,H water are more deformable and can "squeeze" through cell-to-cell connections at the epithelial or epidermal surface and thus achieving deeper layers of the skin or other epithelial linings. This phenomenon positively correlates with lower content of heavy isotopes in water. The penetration of cosmetic compositions with natural water (having relatively high amounts of ¹⁸O and ²H) is much lower than that with water as sued in the cosmetic preparation according to the invention.
If a cosmetic/oral/vaginal composition cannot penetrate epithelial/epidermal barrier, its bioavailability (or capacity to interact with biological processes) is extremely low. In the case of fast and deep penetration, active components of a cosmetic/oral/vaginal composition are highly bioavailable for biological processes going on in the skin or other lining epithelia

### A BRIEF DESCRIPTION OF THE DRAWINGS

Fig. 1 illustrates the absorbance spectrum of verbascoside plus carboxy methyl cellulose in water depleted from ¹⁸O and ²H (De-O,D) after incubation for 30 days;
Fig. 2 illustrates the dependency of superoxide scavenging activity of plant polyphenol (verbascoside) in % over time; and
Fig. 3 is a schematic representation of the production of skin equivalents.

### DETAILED DESCRIPTION OF THE INVENTION

The following examples clarify the invention further in more detail. The depleted water was obtained in all examples by physical purification. Extracts of aquatic and terrestrial plant contain organic molecules, such as lipids, polysaccharides, proteins/amino acids, and polyphenols, which are depleted from heavy hydrogen and oxygen isotopes during their synthesis and isotopic fractionation at definite steps of the biosynthesis Rectification of the water was performed in a rectification column in which water vapor generated in the column still is fed directly to the rectification column where an upward flow of vapor interacts with a downward flow of liquid-reflux.

### Example 1

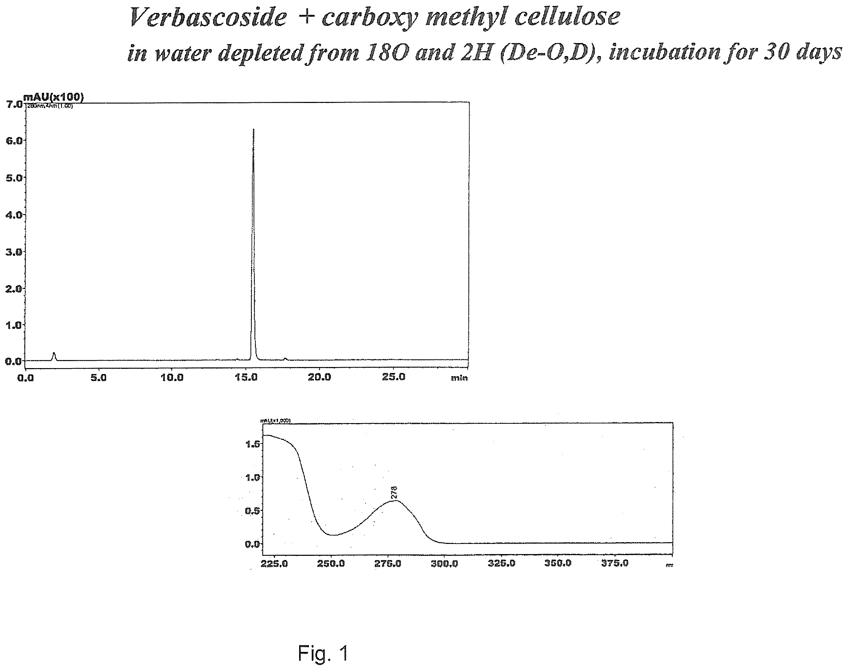

Merystem plant cells containing highly oxydisable active principles such as polyphenols (in this example verbascoside) were homogenized in ¹⁸O and ²H depleted (De-O,D) water having a concentration of 99.894 weight-% (and for comparison in distilled natural water). A hydrogel-forming substance, namely carboxyl methyl cellulose was added to the solutions in a concentration of 0.1 weight-%. The final concentration of verbascoside in the mixture was determined to be 100 µM by HPLC method using chromatographic standard of verbascoside. This concentration corresponds to 6 mg/100 mL or 0.006 weight-%. The maximum of the absorbance spectrum was obtained at 278nm and retention time 15.65 min. The mixture was left in darkness at room temperature (25°C) for thirty days. After this period, the mixture was analyzed for the presence and concentration of verbascoside. The mixture prepared in natural water did not contain any measurable amounts of verbascoside. At the same time, the mixture prepared in ¹⁸O and ²H depleted (De-O,D) water retained practically the initial levels of verbascoside measured by HPLC because the absorbance spectrum and the peak at chromatogram corresponding to verbascoside were only slightly changed (> than 5%) as compared to the initial chromatographic data as shown in figure 1.

### Example 2

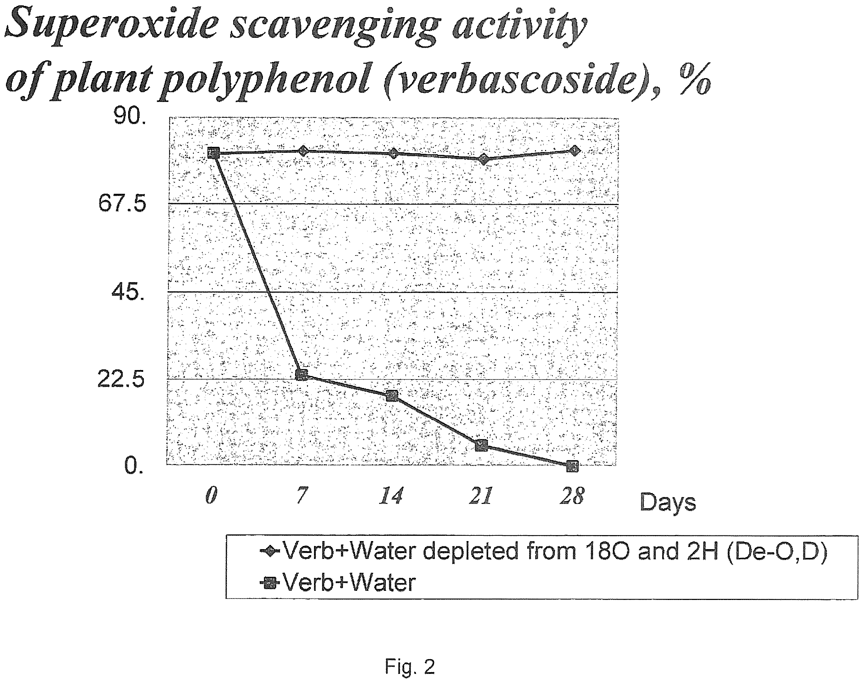

The verbascoside in the mixtures described in Example 1 initially (day 0) possessed superoxide scavenging activity (antioxidant capacity) measured spectrophotometrically by superoxide dismutase dependent reduction of cytochrome c (McCord&Fridovich). In the mixture prepared in natural water of example 1 antioxidant capacity of the polyphenolic molecule verbascoside gradually decayed with the time and was not measurable by day 30 while this capacity remained practically unchanged in the mixture containing De-O,D water, carboxy methyl cellulose and merystem cell homogenate enriched in verbascoside as shown in figure 2.

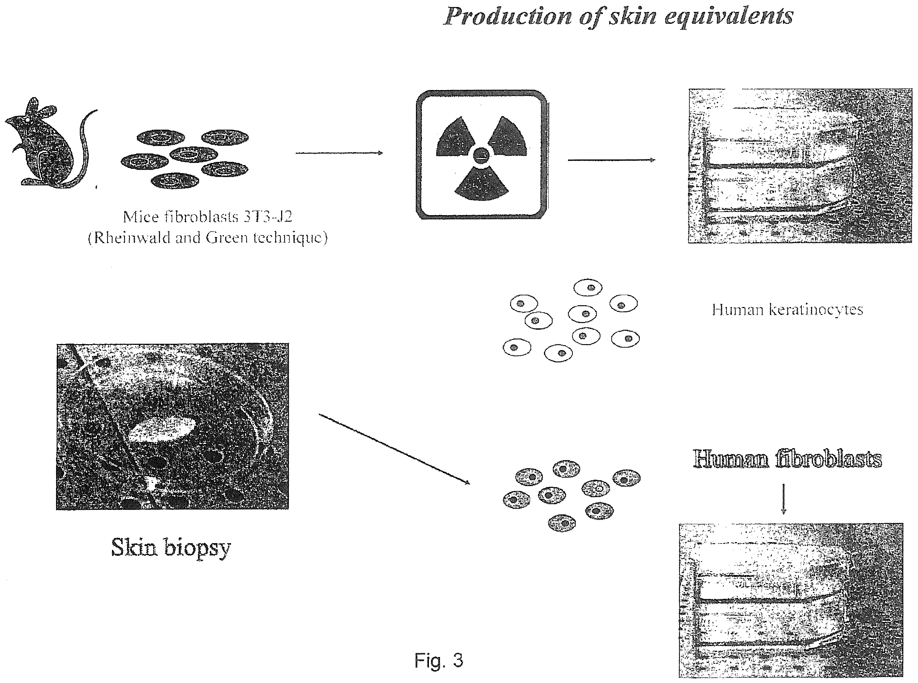

A scheme of preparation of 3D human skin equivalent is represented in figure 3
In tables 1 and 2 the date on anti-inflammatory and UV-protective effects of the composition according to the invention towards 3D human skin equivalent is represented.

### Example 3

Convincing data were obtained on the 3D model of human skin equivalents reconstructed *in vitro* from tiny skin biopsy. The general procedure of the reconstruction is schematically represented in Figure 3.

The treatment with ¹⁸O and ²H depleted (De-O,D) water: Skin equivalents were cultivated in the appropriate medium (Control) or in the same medium containing 20ppm or 50ppm or 80ppm De-O,D water for 7 days when the skin equivalent was formed. Challenge with pro-inflammatory agents: The skin equivalents were exposed to solar simulating UV irradiation containing UVA + UVB (1.0 J/cm2 + 0.1 J/cm2) in doses corresponding to a daily dose of solar irradiation in summer time. Skin samples were processed and examined 6h post-irradiation.

Markers of inflammatory response from human skin cells: The gene expression of TNFalpha, IL-6, and IL-8 as markers of inflammatory response of human skin equivalents was measured by real-time PCR method using molecular primers constructed for each gene. The gene expression of cycloxygenase-2 (COX-2) as a measure of the risk of carcinogenesis was measured by the same approach. Experiments were carried out on three skin cell cultures derived from three different donors. Each measurement was repeated 3-4 times and results were statistically evaluated using the Student's test.

### Results:

- The presence of De-O,D water at 80ppm completely inhibited inflammatory response from human skin cells from TNFalpha and IL-6 induced by solar simulating UVA+UVB irradiation. The presence of De-O,D water at 50ppm was less effective in the inhibition of TNFalpha and IL-6 expression whereas De-O,D water at 20ppm exerted inhibitory efficacy similar to that of 80ppm De-O,D water (Table 1).
- The presence of ¹⁸O and ²H depleted (De-O,D) water at 80ppm partially but substantially (by 30-70%) inhibited the induction of IL-8 and COX-2 gene expression by UV irradiation. At the same time, ¹⁸O and ²H depleted (De-O,D) water at 50ppm and 20ppm was much less effective (Table 1).
- Anti-inflammatory potential of ^{1B}O and ²H depleted (De-O,D) water against UV irradiation is extremely high and comparable with the effects of the most effective anti-inflammatory drugs.

### Example 4

1. Convincing data were obtained on the 3D model of human skin equivalents reconstructed *in vitro* from tiny skin biopsy. The general procedure of the reconstruction is schematically presented in Figure 3.
   The treatment with ¹⁸O and ²H depleted (De-O,D) water: Skin equivalents were cultivated in the appropriate medium (Control) or in the same medium containing 20ppm or 50ppm or 80ppm De-O,D water for 7 days when the skin equivalent was formed. Challenge with bacterial inflammation-inducing agent: The skin equivalents were treated with 1 microg/mL of bacterial lipopolysaccharide (LPS from *E. coli*) to induce inflammatory response of human skin cells to bacterial component.
2. The De-O,D water at 80ppm completely inhibited gene expression of IL-6 and COX-2 induced by LPS while increased the expression of TNFalpha and IL-8 induced by bacterial lipopolysaccharide as shown in table 2..
3. The De-O,D water at 50ppm was more efficient in the inhibition of TNFalpha, IL-6 and COX-2 while less efficient in the induction of IL-8 as shown in table 2.
4. The De-O,D water at 20ppm has the greatest effect on COX-2 inhibition and IL-8 induction.

On the grounds of these data, ¹⁸O and ²H depleted (De-O,D) water with 80-20ppm range from may be useful in the cosmetic and dermatologic preparations (gels, creams, lotions, etc.) with UV-protective, anti-inflammatory, and anti-bacterial claims.

### Determination of deuterium in the finished product

Deuterium content was determined by means of time-based cavity ring-down spectroscopy (CRDS) using a Picarro L2130i/L2140i water isotopic composition analyzer (for D and ¹⁸O) or by means of mass spectroscopy using a Thermo Scientific (Finnigan) series Delta isotope mass spectrometers with an H-Device (Thermo Scientific) peripheral device.

The residual content of deuterium in the final product is expressed in p.p.m. or as a deviation from the International Standard VSMOW (δ), expressed in ppm (‰)

**Table 1. De-O,D water against UV-induced inflammation**

| **Cytokine/System** | **Gene expression** | **Significance** |
|---|---|---|
| **TNFalpha** | | |
| Skin equivalent (SE, control) | 1.02±0.21 | |
| SE+UV | 2.15±0.45* | p<0.01 vs control |
| SE+UV+80ppmDe-O,D | 1.00±0.16** | p<0.01 vs UV |
| SE+UV+50ppmDe-O,D | 1.18+0.17** | p<0.05 vs UV |
| SE+UV+20ppmDe-O,D | 0.95±0.13** | p<0.01 vs UV |

| **IL-6** | | |
|---|---|---|
| SE (control) | 1.00±0.12 | |
| SE+UV | 13.29±2.69* | p<0.01 vs control |
| SE+UV+80ppmDe-O,D | 1.13±0.15** | p<0.01 vs UV |
| SE+UV+50ppmDe-O,D | 2.03±0.21** | p<0.05 vs UV |
| SE+UV+20ppmDe-0,D | 0.90±0.12** | p<0.01 vs UV |

| **COX-2** | | |
|---|---|---|
| SE (control) | 1.00±0.12 | |
| SE+UV | 3.00±0.59* | p<0.01 vs control |
| SE+UV+80ppmDe-O,D | 1.35±0.13** | p<0.01 vs UV |
| SE+UV+50ppmDe-O,D | 1.55±0.13** | p<0.05 vs UV |
| SE+UV+20ppmDe-O,D | 1.83±0.22** | p<0.05 vs UV |

| **Cytokine/System** | **Gene expression** | **Significance** |
|---|---|---|
| **IL-8** | | |
| SE (control) | 1.00±0.00 | |
| SE+UV | 4.23±084* | p<0.01 vs control |
| SE+UV+80ppmDe-O,D | 1.25±0.13** | p<0.01 vs UV |
| SE+UV+50ppmDe-O,D | 2.68±0.35** | p<0.05 vs UV |
| SE+UV+20ppmDe-O,D | 2.75±0.31** | p<0.05 vs UV |

**Table 2. De-O,D water and inflammatory/anti-bacterial responses induced by bacterial LPS**

| **Cytokine/System** | **Gene expression** | **Significance** |
|---|---|---|
| **TNFalpha** | | |
| SE (control) | 1.01±0.03 | |
| SE+UV | 1.23±0.15* | p<0.05 vs control |
| SE+UV+80ppmDe-O,D | 2.45±0.51** | p<0.01 vs LPS |
| SE+UV+50ppmDe-O,D | 2.20±0.52** | p<0.01 vs LPS |
| SE+UV+20ppmDe-O,D | 2.53±0.15** | p<0.01 vs LPS |

| **IL-6** | | |
|---|---|---|
| SE (control) | 1.00±0.12 | |
| SE+UV | 1.67±0.23* | p<0.01 vs control |
| SE+UV+80ppmDe-O,D | 1.00±0.18** | p<0.01 vs LPS |
| SE+UV+50ppmDe-O,D | 0.80±0.18** | p<0.01 vs LPS |
| SE+UV+20ppmDe-O,D | 0.98±0.05** | p<0.01 vs LPS |

| **COX-2** | | |
|---|---|---|
| SE (control) | 1.00±0.12 | |
| SE+UV | 0.88±0.10 | p>0.05 vs control |
| SE+UV+80ppmDe-O,D | 0.70±0.12 | p>0.05 vs LPS |
| SE+UV+50ppmDe-O,D | 0.55±0.13** | p<0.05 vs LPS |
| SE+UV+20ppmDe-O,D | 0.45±0.06** | p<0.05 vs LPS |

| **Cytokine/System** | **Gene expression** | **Significance** |
|---|---|---|
| **IL-8** | | |
| SE (control) | 1.00±0.00 | |
| SE+UV | 1.20±0.16 | p>0.05 vs control |
| SE+UV+80ppmDe-O,D | 4.70±1.10** | p<0.01 vs LPS |
| SE+UV+50ppmDe-O,D | 4.00±0.87** | p<0.01 vs LPS |
| SE+UV+20ppmDe-O,D | 6.13±0.13** | p<0.01 vs LPS |

### Example 5 (body lotion)

59 weight-% of Citrus lemon fruit water with a content of 80 ppm ²H and 1000 ppm of
¹⁸O
7.5 weight-% Persea gratissima oil
5.7 weight-% Aleurites Moluccana seed oil
4.6 weight-% glycerin
23.2 weigth-% mixture of various ingredients used for body lotions.

### Example 6 (creme)

68 weight-% De-O, D water having a content of 50 ppm ²H and 850 ppm of ¹⁸O
9 weight-% cetearyl isononanoate
5 weight-% stearic acid
4 weight-% caprylic triglyceride
14 weigth-% mixture of various ingredients used for cremes

### Example 7 (facial mask)

55 weight-% De-O, D water having a content of 50 ppm ²H and 950 ppm of ¹⁸O
20 weight-% glycerin
15 weight-% propylene glycol
5 weight-% pullulan
5 weight-% mixture of various ingredients used for facial masks

### Example 8 (facial spray)

98.5 weight-% De-O, D water having a content of 45 ppm ²H and 930 ppm of ¹⁸O
0.95 weight-% phenoxyethanol
0.2 weight-% polysorbate 20
0.35 weight-% mixture of various ingredients used for facial sprays.

### Example 9 (hand care)

86.2 weight-% De-O, D water having a content of 75 ppm ²H and 990 ppm of ¹⁸O
5.5 weight-% caprylic triglyceride
2.5 weight-% glyceryl stearate citrate
1.5 weight-% cetearyl alcohol
4.3 weight-% mixture of various ingredients used for hand care

### Example 10 (oral gel)

77.4 weight-% De-O, D water having a content of 48 ppm ²H and 945 ppm of ¹⁸O
10.5 weight-% lactobacillus/papaya fruit ferment extract in an aqueous solution of De-O, D water having a content of 80 ppm ²H and 1000 ppm of ¹⁸O
8 weight-% propylene glycol
3.15 weight-% hydroxypropylmethylcellulose (HPMC)
0.95 weight-% mixture of various ingredients used for oral gels

### Example 11 (serum)

75.7 weight-% De-O, D water having a content of 20 ppm ²H and 750 ppm of ¹⁸O
6 weight-% propylene glycol
3 weight-% pentylene glycol
2.6 weight-% haxapeptide-11
2.46 weight-% methylpropanediol
2.0 weight-% betaine
8.44 weight-% mixture of various ingredients used for serums

### Example 12 (skin aerosol spray)

97.99998706 weight-% De-O, D water having a content of 47 ppm ²H and 945 ppm of ¹⁸O
2 weight-% nitrogen
0.00001294 silver chloride

### Example 13 (vaginal gel)

85.4 weight-% De-O, D water having a content of 55 ppm ²H and 950 ppm of ¹⁸O
10 weight-% lactobacillus/papaya fruit ferment extract in an aqueous solution of De-O, D water having a content of 80 ppm ²H and 1000 ppm of ¹⁸O
3.15 weight-% hydroxypropylmethylcellulose (HPMC)
0.6 weight-% sodium lactate
0.85 weight-% mixture of various ingredients used for vaginal gels.

### Example 14 (veterinary spray)

95.98 weight-% De-O, D water having a content of 80 ppm ²H and 1000 ppm of ¹⁸O
1.98 weight-% PEG-40 hydrogenated castor oil
1.0 weight-% menthol
1.04 weight-% mixture of various ingredients used for veterinary sprays

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

## Claims

1. Cosmetic composition comprising water as a base,
**characterized in that,**
the water contains
a) at least 500 ppm of ¹⁸O and at most 1100 ppm of ¹⁸O; and
b) at least 20 ppm of ²H and at most 90 ppm of ²H.

2. Cosmetic composition according to claim 1, **characterized in that** the water contains
a) at most 1050 ppm of ¹⁸O; and
b) at most 85 ppm of ²H.

3. Cosmetic composition according to claim 2, **characterized in that** the water contains
a) at most 1000 ppm of ¹⁸O; and
b) at most 80 ppm of ²H.

4. Cosmetic composition according to one of the claims 1 to 3, **characterized in that** it comprises water in an amount of 50 to 90 weight-%, preferably 55 to 70 weight-%.

5. Cosmetic composition according to one of the claims 1 to 4, **characterized in that** it is essentially free of alcohols.

6. Cosmetic composition according to one of the claims 1 to 5, **characterized in that** the water is derived from plants comprising substances of cosmetic importance, in particular lipids, polysaccharides, proteins, amino acids and polyphenols, which are depleted from heavy hydrogen and heavy oxygen isotopes during their bio-synthesis and isotopic fractionation at definitive steps of the biosynthesis.

7. Cosmetic composition according to one of the claims 1 to 6, **characterized in that** it further comprises a non-medicinal active principle derived from plant extracts or purified plant-derived agents, in particular polyphenols like flavonoids.

8. Cosmetic composition according to one of the claims 1 to 7, **characterized in that** the water has a weight concentration of residue after evaporation of no more than 5 mg/dm³.

9. Cosmetic composition according to one of the claims 1 to 8, **characterized in that** the water has a weight concentration of KMnO₄(O)-reducing substances of no more than 0.08 mg/dm³.

10. Cosmetic composition according to one of the claims 1 to 9, **characterized in that** the water has a pH value of 5.4 - 6.6.

11. Cosmetic composition according to one of the claims 1 to 10, **characterized in that** the water has a specific conductivity at 20 °C, S/m, of no more than 5·10⁻⁴.

12. Non-therapeutic use of the cosmetic composition according to one of the claims 1 to 11 for topical treatment of ageing human skin and skin appendages, preferably hair or nails or lining epithelia.

13. Composition according to one of claims 1 to 11 for use in the topical treatment of stressed and ailing human skin and skin appendages, preferably hair or nails or lining epithelia.

14. Composition according to one of the claims 1 to 11 for use in topical treatment to attenuate epithelial cell inflammatory responses to biotic and abiotic stresses.

15. Composition according to one of the claims 1 to 11 for use in promoting cellular longevity of skin cells resulting in skin rejuvenation.

16. Cosmetic composition according to one of the claims 1 to 11, **characterized in that** it further comprises a pharmaceutically active drug.

## Patentansprüche

1. Kosmetische Zusammensetzung, umfassend Wasser als Grundlage, **dadurch gekennzeichnet, dass**
das Wasser Folgendes enthält:
a) mindestens 500 ppm von ¹⁸O und höchstens 1100 ppm von ¹⁸O; und
b) mindestens 20 ppm von ²H und höchstens 90 ppm von ²H.

2. Kosmetische Zusammensetzung nach Anspruch 1, **dadurch gekennzeichnet, dass** das Wasser Folgendes enthält:
a) höchstens 1050 ppm ¹⁸O; und
b) höchstens 85 ppm ²H.

3. Kosmetische Zusammensetzung nach Anspruch 2, **dadurch gekennzeichnet, dass** das Wasser Folgendes enthält:
a) höchstens 1000 ppm ¹⁸O; und
b) höchstens 80 ppm ²H.

4. Kosmetische Zusammensetzung nach einem der Ansprüche 1 bis 3, **dadurch gekennzeichnet, dass** sie Wasser in einer Menge von 50 bis 90 Gew.-%, vorzugsweise 55 bis 70 Gew.-% umfasst.

5. Kosmetische Zusammensetzung nach einem der Ansprüche 1 bis 4, **dadurch gekennzeichnet, dass** sie im Wesentlichen frei von Alkoholen ist.

6. Kosmetische Zusammensetzung nach einem der Ansprüche 1 bis 5, **dadurch gekennzeichnet, dass** das Wasser aus Pflanzen, umfassend Substanzen von kosmetischer Bedeutung, insbesondere Lipide, Polysaccharide, Proteine, Aminosäuren und Polyphenole, stammt, die an schweren Wasserstoff- und Sauerstoffisotopen während ihrer Biosynthese und Isotopenfraktionierung in endgültigen Schritten der Biosynthese abgereichert werden.

7. Kosmetische Zusammensetzung nach einem der Ansprüche 1 bis 6, **dadurch gekennzeichnet, dass** sie ferner einen nicht medizinischen Wirkstoff umfasst, der aus Pflanzenextrakten oder gereinigten pflanzlichen Mitteln, insbesondere Polyphenolen wie Flavonoiden, stammt.

8. Kosmetische Zusammensetzung nach einem der Ansprüche 1 bis 7, **dadurch gekennzeichnet, dass** das Wasser eine Gewichtskonzentration des Rückstands nach der Verdampfung von mehr als 5 mg/dm³ aufweist.

9. Kosmetische Zusammensetzung nach einem der Ansprüche 1 bis 8, **dadurch gekennzeichnet, dass** das Wasser eine Gewichtskonzentration von KMnO₄(O)-reduzierenden Substanzen von nicht mehr als 0,08 mg/dm³ aufweist.

10. Kosmetische Zusammensetzung nach einem der Ansprüche 1 bis 9, **dadurch gekennzeichnet, dass** das Wasser einen pH-Wert von 5,4-6,6 aufweist.

11. Kosmetische Zusammensetzung nach einem der Ansprüche 1 bis 10, **dadurch gekennzeichnet, dass** das Wasser eine spezifische Leitfähigkeit bei 20 °C, S/m, von mehr als 5.10⁻⁴ aufweist.

12. Nicht therapeutische Verwendung der kosmetischen Zusammensetzung nach einem der Ansprüche 1 bis 11 für die topische Behandlung von alternder menschlicher Haut und Hautanhangsgebilden, vorzugsweise Haaren oder Nägeln oder Auskleidungsepithel.

13. Zusammensetzung nach einem der Ansprüche 1 bis 11 zur Verwendung bei der topischen Behandlung von beanspruchter und schmerzender menschlicher Haut und Hautanhangsgebilden, vorzugsweise Haaren oder Nägeln oder Auskleidungsepithel.

14. Zusammensetzung nach einem der Ansprüche 1 bis 11 zur Verwendung bei der topischen Behandlung, um Entzündungsreaktionen von Epithelzellen auf biotischen und abiotischen Stress abzuschwächen.

15. Zusammensetzung nach einem der Ansprüche 1 bis 11 zur Verwendung bei der Unterstützung der zellulären Langlebigkeit von Hautzellen, was zu einer Hautverjüngung führt.

16. Kosmetische Zusammensetzung nach einem der Ansprüche 1 bis 11, **dadurch gekennzeichnet, dass** sie ferner einen pharmazeutisch aktiven Wirkstoff umfasst.

## Revendications

1. Composition cosmétique comprenant de l'eau en tant que base,
**caractérisée en ce que**,
l'eau contient
a) au moins 500 ppm de ¹⁸O et au plus 1 100 ppm de ¹⁸O ; et
b) au moins 20 ppm de ²H et au plus 90 ppm de ²H.

2. Composition cosmétique selon la revendication 1, **caractérisée en ce que** l'eau contient
a) au plus 1 050 ppm de ¹⁸O ; et
b) au plus 85 ppm de ²H.

3. Composition cosmétique selon la revendication 2, **caractérisée en ce que** l'eau contient
a) au plus 1 000 ppm de ¹⁸O ; et
b) au plus 80 ppm de ²H.

4. Composition cosmétique selon l'une des revendications 1 à 3, **caractérisée en ce qu'**elle comprend de l'eau dans une quantité de 50 à 90 % en poids, de préférence 55 à 70 % en poids.

5. Composition cosmétique selon l'une des revendications 1 à 4, **caractérisée en ce qu'**elle est essentiellement dépourvue d'alcools.

6. Composition cosmétique selon l'une des revendications 1 à 5, **caractérisée en ce que** l'eau est issue de plantes comprenant des substances d'importance cosmétique, en particulier des lipides, polysaccharides, protéines, acides aminés et polyphénols, qui sont appauvries en isotopes d'hydrogène lourd et d'oxygène lourd pendant leur biosynthèse et fractionnement isotopique lors d'étapes définitives de la biosynthèse.

7. Composition cosmétique selon l'une des revendications 1 à 6, **caractérisée en ce qu'**elle comprend en outre un principe actif non médicinal issu d'extraits de plantes ou d'agents dérivés de plantes, en particulier de polyphénols comme des flavonoïdes.

8. Composition cosmétique selon l'une des revendications 1 à 7, **caractérisée en ce que** l'eau a une concentration en poids de résidu après évaporation de pas plus de 5 mg/dm³.

9. Composition cosmétique selon l'une des revendications 1 à 8, **caractérisée en ce que** l'eau a une concentration en poids de substances réductrices KMnO₄(O) de pas plus de 0,08 mg/dm³.

10. Composition cosmétique selon l'une des revendications 1 à 9, **caractérisée en ce que** l'eau a une valeur de pH de 5,4 à 6,6.

11. Composition cosmétique selon l'une des revendications 1 à 10, **caractérisée en ce que** l'eau a une conductivité spécifique à 20 °C, S/m, de pas plus de 5.10⁻⁴.

12. Utilisation non thérapeutique de la composition cosmétique selon l'une des revendications 1 à 11 pour un traitement topique de la peau humaine vieillissante et des annexes cutanées, de préférence les cheveux ou les poils ou les ongles ou les épithéliums de revêtement.

13. Composition selon l'une des revendications 1 à 11, pour utilisation dans le traitement topique de la peau humaine stressée et malade et des annexes cutanées, de préférence les cheveux ou les poils ou les ongles ou les épithéliums de revêtement.

14. Composition selon l'une des revendications 1 à 11, pour utilisation dans le traitement topique pour atténuer des réponses inflammatoires de cellules épithéliales à des stress biotiques et abiotiques.

15. Composition selon l'une des revendications 1 à 11, pour utilisation dans la promotion de longévité cellulaire de cellules de la peau conduisant à un rajeunissement de la peau.

16. Composition cosmétique selon l'une des revendications 1 à 11, **caractérisée en ce qu'**elle comprend en outre un médicament pharmaceutiquement actif.
